# EUROPEAN PATENT APPLICATION

(11) **EP 4 098 205 A1**
(43) Date of publication of application: **07.12.2022**
(21) Application number: 21180055.2
(22) Date of filing: 17.06.2021
(51) Int. Cl.: A61B 8/08, A61B 8/00

(54) **POWER REDUCTION IN ULTRASOUND SYSTEMS**

(30) Priority: 31.05.2021 US 202163195073 P
(71) Applicant: Koninklijke Philips N.V., 5656 AG Eindhoven (NL)
(72) Inventor: WILSON, Martha Gail Grewe, 5656 AE Eindhoven (NL); POLAND, McKee Dunn, 5656 AE Eindhoven (NL); RAJU, Balasundar Iyyavu, 5656 AE Eindhoven (NL)
(74) Representative: Philips Intellectual Property & Standards

(57) **Abstract**

The invention provides a system for adapting operating parameters of an ultrasound scanner, the system comprising a processing arrangement configured to obtain ultrasound data of a subject from the ultrasound scanner based on a plurality of operating parameters and detect a feature of interest in the ultrasound data using a feature detection algorithm. The feature of interest for example comprises an organ, vessel or bone of interest; a region of interest of an organ, vessel or bone; or a pathology of interest. The processing arrangement is configured to adapt the operating parameters based on whether the feature of interest has been detected.

## Description

### FIELD OF THE INVENTION

The invention relates to ultrasound scanning, such as for medical imaging. In particular, the invention relates to the operating parameters of ultrasound scanners.

### BACKGROUND OF THE INVENTION

Ultrasonic transducers convert an alternating current (AC) drive signal into an ultrasound pressure wave, as converting a received pressure wave into an electrical signal. Piezoelectric crystals change size and shape when a voltage is applied. An AC voltage makes them oscillate at the same frequency and produce ultrasonic sound. Capacitive transducers for example use electrostatic fields between a conductive diaphragm and a backing plate.

Since piezoelectric materials generate a voltage when force is applied to them, they can also work as ultrasonic detectors. Some systems use separate transmitters and receivers, while others combine both functions into a single piezoelectric transceiver.

A capacitor microphone has a thin diaphragm that responds to ultrasound waves. Changes in the electric field between the diaphragm and a closely spaced backing plate convert sound signals to electric currents, which can be amplified. The stronger the transmitted ultrasound, the more which can be detected after it has been reflected.

Thus, it is clear the amount of data which can be obtained from an ultrasound system is linked to the power drawn by the ultrasound system. One of the challenges with typical ultrasound systems is the amount of power required to drive the transducer without overheating. With more of the system functionality added into the transducer head and with the possibility of three dimensional (3D) imaging, power management continues to be an important part of the design.

Thus, there is a need to enable high power delivery to ultrasound scanners in order to ensure high quality data, but to reduce the overall power consumption.

### SUMMARY OF THE INVENTION

The invention is defined by the claims.

According to examples in accordance with an aspect of the invention, there is provided a system for adapting operating parameters of (in other words, for configuring) an ultrasound scanner, the system comprising:
a first processor configured to:
obtain ultrasound data of a subject from an ultrasound scanner based on a plurality of operating parameters;
detect a feature of interest in the ultrasound data, using a feature detection algorithm; and
adapt the operating parameters based on whether the first processor has detected the feature of interest.

The adapted operating parameters may permit the ultrasound scanner to draw a relatively high maximum power and the non-adapted operating parameters limit the ultrasound scanner to draw a low maximum power.

For example, the amount of power required to operate portable and hand-held ultrasound scanners can cause overheating over time. In order to avoid overheating, the scanner is typically operated at lower qualities in order to reduce the power draw from the scanner and increase the time over which it can be operated. However, reducing the quality of data obtained by the scanner also reduces the usefulness of the images generated in the case of a medical imaging system.

The power draw of an ultrasound scanner is typically dependent on the operating parameters chosen. The operating parameters also dictate the quality of the ultrasound data Thus, the inventors propose using a "feature detection algorithm" (e.g. using a first processor) to detect features in ultrasound data and adapting the operating parameters (e.g. using a second processor) based on a feature being found.

For example, the operating parameters may be adapted in order to improve the quality of the ultrasound data when a feature has been found. In this case, the ultrasound scanner could be used with operating parameters which reduce the power draw, and thus allow the scanner to be used for longer without overheating.

The feature of interest for example comprises:
an organ, vessel or bone of interest;
a region of interest of an organ, vessel or bone; or
a pathology of interest.

The processing arrangement may comprise a first processor to detect the feature of interest and a second processor to perform the adaptation of the operating parameters.

The first processor is able to detect features in the ultrasound data at lower qualities. Once a feature is found, the operating parameters can be adapted to improve the quality of the ultrasound data.

The ultrasound data may be ultrasound images.

In some examples, the first and second processors may be part of a processing unit. The first processor may also be the second processor (i.e. the same processor is used for all of the steps).

Alternatively, the first processor may be located outside of the ultrasound scanner and the second processor may be located within the ultrasound scanner. The first and second processors may communicate wirelessly (e.g. WiFi, Bluetooth etc.).

Pathologies of interest may include, for example: cancer or tumors somewhere in the body, lung pathologies (e.g. lung consolidation, pleural effusions, and air bronchograms), plaque or thrombus/obstructions in blood vessels (i.e. narrowing of arteries), liver pathologies (e.g. steatosis and cysts), ovarian cysts, gall stones, aneurysms, injuries or alterations to any organ or tissue, thyroid cysts, hematoma due to trauma, bleeds /free fluid in the abdomen or chest, tendon tears or bladder masses.

The system may further comprise a power management sub-system for providing power to the ultrasound scanner, wherein the operating parameters are limited by a maximum power drawn by the ultrasound scanner.

Adapting the operating parameters may comprise applying a low power mode to the ultrasound scanner, wherein the low power mode comprises configuring the ultrasound scanner to draw a low maximum power from a power management sub-system based on the feature of interest not being detected in the ultrasound data, and applying a high power mode to the ultrasound scanner, wherein the high power mode comprises configuring the ultrasound scanner to draw a high maximum power from the power management sub-system based on the feature of interest being detected in the ultrasound data.

Limiting the power drawn by the ultrasound scanner also limits the heat output of the ultrasound scanner, thus increasing the time it takes for the ultrasound scanner to overheat. This allows the ultrasound scanner to be used for longer when searching for a feature of interest (i.e. anatomical part or pathology). When the feature is found, the maximum power which can be drawn by the ultrasound scanner is increased which enables the ultrasound data to be generated at a higher quality.

The particular configuration of the low power mode may be pre-selected by a user or automatically selected by the processors. The particular high power mode may be chosen from a pre-selected list or generated on the spot based on the ultrasound data, the feature of interest and/or an indication of confidence.

Detecting a feature of interest in the ultrasound data may be performed by an inference engine.

In the field of artificial intelligence, an inference engine is a component of a system that applies logical rules to a knowledge base to deduce new information. For example, the ultrasound data could be input into the inference engine in order to infer the presence of an organ based on the patterns and the acoustic characteristics in the ultrasound data.

Alternatively, detecting a feature of interest in the ultrasound data may be performed by a heuristic algorithm.

A heuristic function is based on a technique designed for solving a problem quickly when classic methods are too slow, or for finding an approximate solution when classic methods fail to find any exact solution. This is achieved by trading optimality, completeness, accuracy, or precision for speed. A heuristic function may allow ultrasound data to be analyzed for each frame in the feed from an ultrasound scanner (e.g. 20 frames per second).

The operating parameters may comprise one or more of: a transmit pulse power, a scan beam spacing, a scan line duration, a scan line density, a signal processing rate and an image display rate.

The first processor may be further configured to output an indication of confidence, wherein the indication of confidence indicates the likelihood of the detected feature of interest being present in the ultrasound data.

For example, the likelihood of confidence may be a number in a range from zero to one. A value of one may indicate that the feature is certainly in the ultrasound data and a value of zero may indicate that the feature is certainly not in the ultrasound data.

The second processor may be configured to adapt the operating parameters further based on the indication of confidence.

For example, the "optimal" or "best" operating parameters may only be used if the indication of confidence is above 0.8 (within the total range 0 to 1). This allows the ultrasound scanner to be used for longer before overheating when the presence of a feature is relatively uncertain.

The system may further comprise an audio system configured to provide an audio cue based on the first processor detecting the feature of interest.

The system may further comprise a user input for receiving, from a user, identification of the feature of interest to be detected by the first processor in the ultrasound data.

The system may further comprise a memory storing a pre-selected set of a plurality of features of interest, wherein the first processor is configured to detect any one of the features of interest.

There is also proposed an ultrasound scanning device which comprises the system for adapting operating parameters of an ultrasound scanner, as mentionned above, and an ultrasound scanner. The ultrasound scanner may be a portable or handheld ultrasound scanner. The ultrasound scanner may comprise one or more ultrasound transducers. The ultrasound scanner may take the form of a probe, and may have any form factor suitable to be held on the surface of a volume or region to be scanned by means of emitted and received ultrasound signals.

The invention also provides a method for adapting operating parameters of an ultrasound scanner, comprising:
obtaining ultrasound data of a subject from an ultrasound scanner based on a plurality of operating parameters;
detecting a feature of interest in the ultrasound data; and
adapting the operating parameters based on whether the feature of interest has been detected.

Adapting the operating parameters may comprise applying a low power mode to the ultrasound scanner, wherein the low power mode comprises configuring the ultrasound scanner to draw a low maximum power from a power management sub-system based on the feature of interest not being detected in the ultrasound data, and applying a high power mode to the ultrasound scanner, wherein the high power mode comprises configuring the ultrasound scanner to draw a high maximum power from the power management sub-system based on the feature of interest being detected in the ultrasound data.

The method may further comprise outputting an indication of confidence, wherein the indication of confidence indicates the likelihood of the detected feature of interest being present in the ultrasound data.

Adapting the operating parameters may be further based on the indication of confidence.

The invention also provides computer program product comprising a computer readable medium, the computer readable medium having computer readable code embodied therein, the computer readable code being configured such that, on execution by a suitable computer or processor, the computer or processor is caused to perform all of the steps of the aforementioned method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an exemplary ultrasound system;
Fig. 2 shows a method for switching from a low power mode to a high power mode; and
Fig. 3 shows a method for adapting the operating parameters of an ultrasound scanner.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for adapting the operating parameters of an ultrasound scanner, the system comprising a processor arrangement configured to obtain ultrasound data of a subject from an ultrasound scanner based on a plurality of operating parameters and detect a feature of interest in the ultrasound data using a feature detection algorithm. The feature of interest for example comprises an organ, vessel or bone of interest; a region of interest of an organ, vessel or bone; or a pathology of interest. The processor arrangement is further configured to adapt the operating parameters based on whether the first processor has detected the feature of interest. The invention is of particular interest for medical imaging.

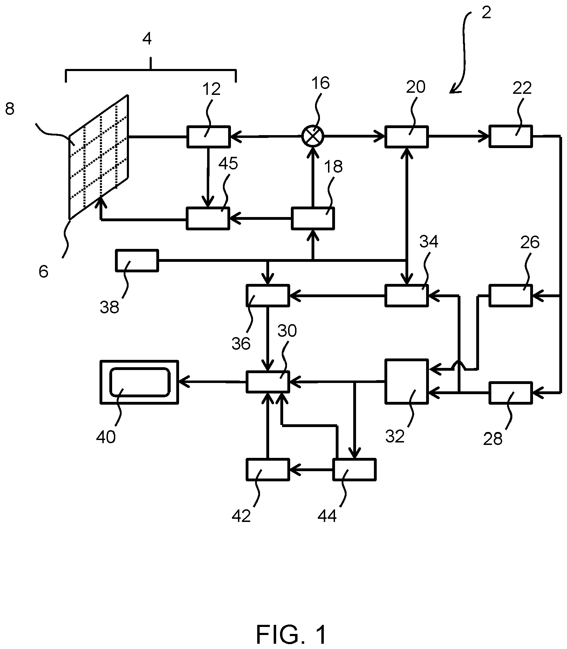

The general operation of an exemplary ultrasound scanner (i.e. ultrasound system) will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display unit 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

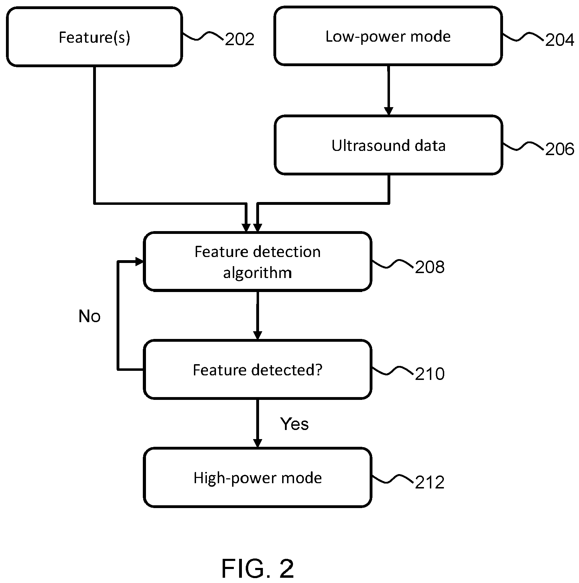

Fig. 2 shows a method for switching from a low power mode 204 to a high power mode 212. The user might select a particular feature of interest 202 from a set of features from, for example, a drop-down list. Alternatively, the feature 202 to be detected may be auto-selected by the processor based on scan presets. Several examples of features of interest 202 are mentioned below:
- Liver-kidney organ identification for an abdominal exam;
- Apical four-chamber or parasternal views for cardiac exam;
- Inferior vena cava (IVC) vessel identification for fluid response assessment;
- Bladder organ identification for bladder volume assessment;
- Mid-cerebral artery identification for transcranial imaging; and
- Peripheral vessel identification for vascular access.

The feature 202 being detected could be a pathology instead of an anatomical feature. In such as case, the user might provide an override to stop the ultrasound scanner from scanning when the pathology is not detected after a pre-determined time.

Pathologies of interest may include, for example: cancer or tumors somewhere in the body, lung pathologies (e.g. lung consolidation, pleural effusions, and air bronchograms), plaque or thrombus/obstructions in blood vessels (i.e. narrowing of arteries), liver pathologies (e.g. steatosis and cysts), ovarian cysts, gall stones, aneurysms, injuries or alterations to any organ or tissue, thyroid cysts, hematoma due to trauma, bleeds /free fluid in the abdomen or chest, tendon tears or bladder masses.

The ultrasound scanner starts operation with a low power mode 204. This could be achieved using one of several means. The low power mode 204 ensures the ultrasound scanner can only draw a relatively low power for obtaining the ultrasound data 206 by limiting the operating parameters of the ultrasound scanner. For example, a 3D ultrasound scanner could use sparser planes, lower line density, lower acoustic transmit power settings, lower frame rate, or a combination thereof.

A feature detection algorithm is invoked on the ultrasound data collected by the ultrasound scanner. The feature detection algorithm 208 looks for whether the particular feature 202 is identified. For example, in a cardiac imaging application, this could be the identification of an apical four-chamber view. The identification of the feature 202 may be based on the feature detection algorithm 208 outputting an indication of confidence (i.e. a confidence score) where a pre-determined threshold confidence score indicates that the entire heart is present within the ultrasound data 206 (e.g. a 3D volume of interest).

The feature detection algorithm 208 could employ artificial intelligence methods such as object detection with a confidence score generated based on whether an object is present within the ultrasound data 206 (e.g. if the object is present in an ultrasound image). Feature detection may be confirmed once the confidence score is above the pre-determined threshold confidence score.

Other techniques based on convolutional neural networks for image classification may also be employed for the feature detection algorithm 208. Furthermore, methods based on conventional image analysis (e.g. correlation analysis, template matching, spatial frequency analysis, temporal domain analysis, spectral analysis, etc.) may also be used.

In the examples above, analysis of image data is used to identify the feature of interest. However, non-image data may instead be used, such as raw RF data from an ultrasound scanner, and spectral analysis for example may be used to identify features of interest.

In step 210, if the feature 202 is not detected, the ultrasound scanner stays in the low power mode 204 and the feature detection algorithm 208 continues processing the ultrasound data 206.

Once the feature 202 is determined using the low power mode 204, the processor enables a high power mode 212 for the ultrasound scanner. The high power mode 212 enables the ultrasound scanner to obtain higher quality ultrasound data that is better suited for data analysis (e.g. quantification) or more accurate pathology classification.

The high power mode 212 could be enabled by increasing the scan line density, increased number of scan planes (for 3D ultrasound scanner), higher acoustic transmit power, higher framerate, or a combination thereof. The high power mode 212 then enables a richer set of data ideal for data analysis. Any number of algorithms may be invoked to perform the necessary quantification or assessment on the high quality ultrasound data. Examples of such assessments include the following:
- Left ventricle volume calculation
- Volume of free fluid in an abdominal exam
- Collapsibility of the inferior vena cava for fluid status assessment
- Bladder volume calculation

Switching to the high power mode 212 after a feature 202 is detected addresses the issue of overheating while addressing the clinical need for maximum acoustic power at the spot of the surveillance. Since the controls are automated, this can be done 'under the hood' and quickly. The low power mode 204 during surveillance results in not only a lower acoustic power transmitted, but also lowers the computational power required for the feature detection algorithm 208.

The ultrasound scanner might periodically cycle between the low power mode 204 and the high power mode 212. Initially the low power mode 204 is used for gross feature identification, after which the processors switch the ultrasound scanner to the high power mode 212 for analysis of the high accuracy ultrasound data. Subsequently the ultrasound scanner may be turned back to the low power mode 204 so as to preserve power and then switch to the high power mode 212 when the ultrasound data 206 changes substantially. The cycle may repeat multiple times.

The lower power mode 204 may comprise one or more low power sub-modes for different situations. Similarly, the high power mode 212 may comprise one of more high power sub-modes for different situations.

Optionally, an audio cue may be provided to the user to keep the ultrasound scanner still once the low power mode 204 has identified the feature of interest 202.

This method requires the ultrasound scanner to interact with the processor arrangement (e.g. first and second processors!) detecting the feature of interest 202. The processors may interact with an artificial intelligence acquisition assistance algorithm (to assist the feature detection algorithm 208) and/or a power management sub-system (to switch between the low power mode 204 and the high power mode 212).

The ultrasound scanner may initiate the analysis automatically. Advantageously the ultrasound scanner may provide an audible beep to the user to hold the probe steady and may ask for a user confirmation prior to the analysis.

While the use of low power mode 204 and high power mode 212 is particularly useful for a 3D ultrasound scanner, it is also useful for a 2D ultrasound scanner when operated by a battery powered system. This is especially useful in an ultra-low form-factor device where the ultrasound scanner is used as a sensor with no images or very coarse images shown on a display unit. Here, an inference engine could evaluate features 202 that are of interest in the 2D image in a low power mode 204, and then switch to a high power mode 212 when a feature 202 is recognized. The low power mode 204 could involve sparse set of lines.

Optionally, the ultrasound scanner is operated with different line densities at different scan planes such that the plane displayed is rendered based off of high line density data, but the other image data is in a low line density mode and not rendered. The non-rendered planes can be used to make inference on the anatomical feature and then guide user to manipulate the probe accordingly.

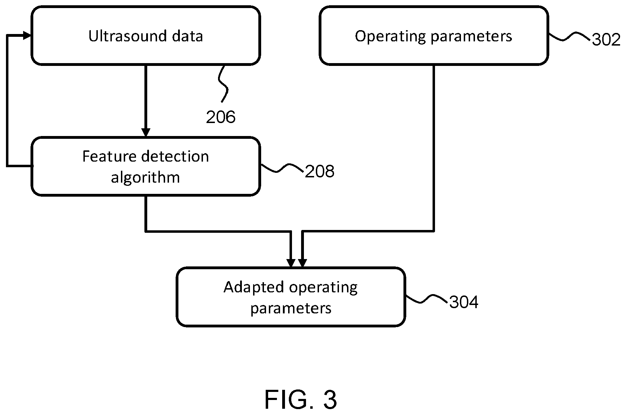

Fig. 3 shows a method for adapting the operating parameters 302 of an ultrasound scanner. The method comprises obtaining ultrasound data 206 of a subject from an ultrasound scanner. The quality of the ultrasound data 206 is directly correlated to the operating parameters 302 used by the ultrasound scanner. For example, the initial operating parameters enable the ultrasound scanner to obtain low quality ultrasound data 206.

A feature of interest 202 is then detected in the ultrasound data 206 using a feature detection algorithm 208. The feature of interest 202 comprises a particular organ, vessel or bone of interest; a region of interest of an organ, vessel or bone; or a pathology of interest.

The feature of interest 202 does not contain solely the type of tissue to be detected. Solely defining the type of tissue does not indicate a feature 202, it merely indicates the possibility of finding a feature 202 in the vicinity. For example, detecting bone in the ultrasound data 206 does not define which bone it is.

Once a feature 202 has been detected, the operating parameters 302 are adapted. For example, the adapted operating parameters 304 enable the ultrasound scanner to obtain high quality ultrasound data.

Higher quality ultrasound data is not required when merely searching for a feature 202 and may cause overheating of the ultrasound scanner. Thus, the operating parameters 302 are initially kept relatively low whilst searching for the feature 202 and are adapted when the feature 202 is detected in order to obtain higher quality ultrasound data of the feature 202.

The skilled person would be readily capable of developing the processor arrangement for carrying the methods described above. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for adapting operating parameters of an ultrasound scanner, the system comprising:
a processor arrangement configured to:
obtain ultrasound data (206) of a subject from an ultrasound scanner based on a plurality of operating parameters (302);
detect a feature of interest (202) in the ultrasound data (206) using a feature detection algorithm (208); and
adapt the operating parameters (302) based on whether the feature of interest has been detected (202).

2. The system of claim 1, wherein feature of interest (202) comprises:
an organ, a vessel or a bone of interest;
a region of interest of an organ, a region of interest of a vessel or a region of interest of a bone; or
a pathology of interest.

3. The system of claim 1 or 2, further comprising a power management sub-system for providing power to the ultrasound scanner, wherein the operating parameters (302) are limited by a maximum power drawn by the ultrasound scanner and wherein adapting the operating parameters (302) comprises:
applying a low power mode (204) to the ultrasound scanner based on the feature of interest (202) not being detected in the ultrasound data (206), wherein applying the low power mode (204) comprises configuring the ultrasound scanner to draw a low maximum power from the power management sub-system; and
applying a high power mode (212) to the ultrasound scanner based on the feature of interest (202) being detected in the ultrasound data (206), wherein applying the high power mode (212) comprises configuring the ultrasound scanner to draw a high maximum power from the power management sub-system,
wherein the high maximum power is higher than the low maximum power.

4. The system of any one of claims 1 to 3, wherein detecting a feature of interest (202) in the ultrasound data (206) is performed by an inference engine or by a heuristic algorithm.

5. The system of any one of claims 1 to 4, wherein the operating parameters (302) comprise one or more of:
a transmit pulse power;
a scan beam spacing;
a scan line duration;
a scan line density;
a signal processing rate; and
an image display rate.

6. The system of any one of claims 1 to 5, wherein the processing arrangement is further configured to output an indication of confidence, wherein the indication of confidence indicates the likelihood of the detected feature of interest (202) being present in the ultrasound data (206), and wherein the processing arrangement is configured to adapt the operating parameters (302) further based on the indication of confidence.

7. The system of any one of claims 1 to 6, further comprising an audio system configured to provide an audio cue based on the processing arrangement detecting the feature of interest (202).

8. The system of any one of claims 1 to 7, further comprising a user input for receiving, from a user, identification of the feature of interest (202) to be detected by the processing arrangement in the ultrasound data (206).

9. The system of any one of claims 1 to 7, further comprising a memory storing a pre-selected set of a plurality of features of interest, wherein the processing arrangement is configured to detect any one of the features of interest.

10. An ultrasound scanning device, comprising:
an ultrasound scanner and
the system as claimed of any one of claims 1 to 10.

11. A method for adapting operating parameters of an ultrasound scanner, comprising:
obtaining ultrasound data (206) of a subject from an ultrasound scanner based on a plurality of operating parameters (302);
detecting a feature of interest (202) in the ultrasound data (206); and
adapting the operating parameters (302) based on whether the feature of interest (202) has been detected.

12. The method of claim 11, wherein feature of interest (202) comprises:
an organ, vessel or bone of interest;
a region of interest of an organ, vessel or bone; or
a pathology of interest.

13. The method of claim 11 or 12, wherein the operating parameters (302) are limited by a maximum power drawn by the ultrasound scanner and wherein adapting the operating parameters (302) comprises:
applying a low power mode (204) to the ultrasound scanner based on the feature of interest (202) not being detected in the ultrasound data (206), wherein applying the low power mode (204) comprises configuring the ultrasound scanner to draw a low maximum power; and
applying a high power mode (212) to the ultrasound scanner based on the feature of interest (202) being detected in the ultrasound data (206), wherein applying the high power mode (212) comprises configuring the ultrasound scanner to draw a high maximum power,
wherein the high maximum power is higher than the low maximum power.

14. The method of any one of claims 11 to 13, further comprising outputting an indication of confidence, wherein the indication of confidence indicates the likelihood of the detected feature of interest (202) being present in the ultrasound data (206) and wherein adapting the operating parameters (302) is further based on the indication of confidence.

15. A computer program product comprising a computer readable medium, the computer readable medium having computer readable code embodied therein, the computer readable code being configured such that, on execution by a suitable computer or processor, the computer or processor is caused to perform the method as claimed in any of claims 11 to 14.
